# EUROPÄISCHE PATENTSCHRIFT

(11) **EP 0 434 832 B1**
(45) Veröffentlichungstag und Bekanntmachung des Hinweises auf die Patenterteilung: **04.05.1994**
(21) Anmeldenummer: 90917918.6
(22) Anmeldetag: 13.07.1990
(51) Int. Cl.: A61K 31/557, C07C 405/00

(54) **D8- und D9-PROSTAGLANDIN-DERIVATE, VERFAHREN ZU IHRER HERSTELLUNG UND IHRE PHARMAZEUTISCHE VERWENDUNG**
D8- and D9-PROSTAGLANDINE DERIVATES, THEIR PROCESS OF PRODUCTION AND THEIR PHARMACEUTICAL USE
DERIVES DE D8- et de D9-PROSTAGLANDINE, LEUR PROCEDE DE PRODUCTION ET LEUR UTILISATION PHARMACEUTIQUE

(30) Priorität: 14.07.1989 DE 3923798
(43) Veröffentlichungstag der Anmeldung: 03.07.1991
(73) Patentinhaber: SCHERING AKTIENGESELLSCHAFT, 13342 Berlin (DE)
(72) Erfinder: KLAR, Ulrich, D-1000 Berlin 27 (DE); REHWINKEL, Hartmut, D-1000 Berlin 44 (DE); VORBRÜGGEN, Helmut, D-1000 Berlin 27 (DE); THIERAUCH, Karl-Heinz, D-1000 Berlin 37 (DE); STÜRZEBECHER, Claus-Steffen, D-1000 Berlin 41 (DE)
(86) Internationale Anmeldenummer: DE9000541
(87) Internationale Veröffentlichungsnummer: WO9101303

(56) Entgegenhaltungen:
- EP-A- 0 098 141
- EP-A- 0 172 963
- DE-A- 2 550 436
- DE-A- 2 757 834
- DE-A- 3 125 271
- US-A- 4 142 052

## Beschreibung

Die Erfindung betrifft Δ⁸- -Prostaglandin-Derivate, Verfahren zu ihrer Herstellung sowie ihre Verwendung als Hilfsstoffe für pharmakologische Untersuchungen und als Arzneimitel.

Aus DE-A-25 50 436 und DE-A-27 57 834 sind 16-Aryloxy- und Arylthio-Δ⁹-Prostaglandin-Derivate bekannt. In der Offenlegungsschrift DE-A-31 25 271 werden Aryloxy- und Arylthio-Δ⁸-Prostaglandin-Derivate mit luteolytischer und abortiver Wirkung beschrieben.

Es wurde überraschenderweise gefunden, daß durch die Einführung einer Doppelbindung in die Position 8 in Verbindung mit einem aromatischen Substituenten an Position 17 chemisch und metabolisch stabile Prostaglandin-Analoga erhalten werden, deren pharmakologische Eigenschaften mit denen des instabilen Thromboxan-A₂(TXA₂) bzw. PGH ₂ vergleichbar sind.
Die Verbindungen dieser Erfindung eignen sich deshalb als Hilfsmittel für pharmakologische Charakterisierungen sowie zur selektiven Therapie von Erkrankungen, die auf einen Mangel an körpereigenem TXA₂/PGH₂ zurückzuführen sind.

Die Erfindung betrifft Δ⁸- -Prostaglandin-Derivate der Formel I, 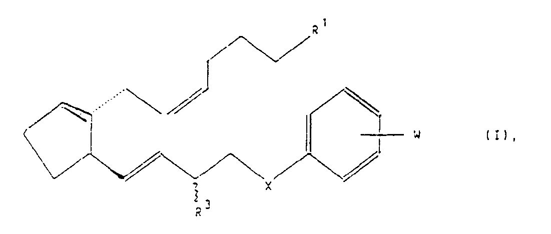
worin 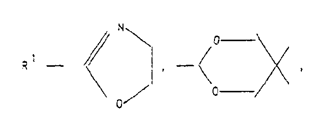
COOR⁴, wobei R⁴ wasserstoff
oder einen gegebenenfalls durch Halogen, Phenyl, C₁-C₄ -Alkoxy oder Di-(C₁-C₄)-alkylamino substituierten C₁-C₁₀-Alkyl-Rest, einen C₃-C₁₀-Cycloalkyl-, einen C₇-C₁₆-Aralkyl-, einen durch W substituierten Phenacyl-Rest, einen C₆-C₁₂-Aryl- Rest oder einen 5- oder 6-gliedrigen heterocyclischen Rest mit wenigstens einem N-, O- oder S-Atom bedeuten kann, oder R¹ ein CONHR⁵-Rest mit R⁵ in der Bedeutung Wasserstoff, C₁-C₁₀-Alkanoyl oder C₁-C₁₀-Alkansulfonyl sein kann,
R³ ein Wasserstoffatom oder eine freie oder funktionell abgewandelte Hydroxygruppe, wobei die OH-Gruppe jeweils α- oder β-ständig sein kann, bedeuten,
- X: eine CH₂-Gruppe, ein O- oder S-Atom,
- W: Wasserstoff, -OR⁶, Halogen, -CN-,-NO₂, Trifluormethyl oder COOR⁶,
- R⁶: Wasserstoff, C₁-C₁₀-Alkyl, durch Halogen substituiertes C₆-C₁₂-Aryl oder C₇-C₁₆-Aralkyl sein kann und, falls R⁴ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

Die Definition 5- oder 6-gliedriger heterocyclischer Rest betrifft Heterocyclen, die wenigstens ein Heteroatom, vorzugsweise Stickstoff, Sauerstoff oder Schwefel enthalten. Beispielsweise seien genannt 2-Furyl, 3-Furyl, 2-Thienyl, 3-Thienyl, 2-Pyridyl, 3-Pyridyl, 4-Pyridyl.
Als Alkylgruppen R⁴ und R⁶ sind gerad- oder verzweigtkettige Alkylgruppen mit 1-10 C-Atomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.

Die Alkylgruppen R⁴ und R⁶ können substituiert sein durch Halogenatome, Hydroxygruppen, C₁-C₄-Alkoxygruppen, C₆-C₁₂-Arylgruppen, die durch Halogen substituiert sein können, Di-(C₁-C₄)-Alkylamine und Tri-(C₁-C₄)-Alkylammonium. Bevorzugt sind solche Alkylgruppen, die einfach substituiert sind.

Als Substituenten seien beispielsweise genannt Fluor-, Chlor- oder Bromatome, Phenyl, Dimethylamino, Diethylamino, Methoxy, Ethoxy.

Als bevorzugte Alkylgruppen R⁴ und R⁶ sind solche mit 1-4 C-Atomen, wie zum Beispiel Methyl, Ethyl, Propyl, Isobutyl, Butyl, zu nennen.

Als Arylgruppen R⁴ und R⁶ kommen beispielsweise in Betracht: Phenyl, Diphenyl, 1-Naphthyl und 2-Naphthyl, die substituiert sein können durch 1-3 Halogenatome, eine Phenylgruppe, 1-3 Alkylgruppen mit jeweils 1-4 C-Atomen, eine Chlormethyl-, Fluormethyl-, Carboxyl-, C₁-C₄-Alkoxy- oder Hydroxygruppe. Bevorzugt ist die Substitution in 3- und 4-Stellung am Phenylring zum Beispiel durch Fluor, Chlor, C₁-C₄-Alkoxy oder Trifluormethyl oder in 4-Stellung durch Hydroxy.

Die Cycloalkylgruppen R⁴ können im Ring 3-10, vorzugsweise 3-6 Kohlenstoffatome enthalten. Die Ringe können durch Alkylgruppen mit 1-4 Kohlenstoffatomen substituiert sein. Beispielsweise seien genannt Cyclopropyl, Cyclobutyl, Cyclopentyl, Methylcyclopentyl, Methylcyclohexyl.
Besonders bevorzugte Cycloalkylgruppen sind Cyclopentyl und Cyclohexyl.
Als C₇-C₁₆-Aralkyl sind folgende Reste gemeint: Phenyl-substituierte Alkylreste (geradkettige und verzweigte) mit 1-10 C-Atomen wie zum Beispiel Benzyl, Phenylmethyl, α-Phenylethyl, 3-Phenylpropyl usw. Als Ar kommen aber auch 1-oder 2-Naphthyl mit entsprechend kürzerer Alkylkette in Betracht.

Die als Substituenten genannten Alkyl- oder Alkoxygruppen mit 1-4 C-Atomen sollen gerad- oder verzweigtkettig sein.

Die Hydroxygruppe in R³ kann funktionell abgewandelt sein, beispielsweise durch Veretherung oder Veresterung, wobei die freien oder abgewandelten Hydroxygruppen α- oder β-ständig sein können, wobei freie Hydroxygruppen bevorzugt sind.

Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, tert.-Butyldimethylsilyl-, tert.-Butyldiphenylsilyl-, Tribenzylsilylrest. Als Acylreste kommen zum Beispiel Acetyl, Propionyl, Butyryl, Benzoyl in Frage.

Halogen in den Definitionen für R⁴, R⁶ und W bedeutet Fluor, Chlor und Brom.

Die Reste "C₁-C₁₀-Alkanoyl" oder "C₁-C₁₀-Alkansulfonyl" für R⁵ entsprechen den bereits genannten Alkylgruppen gleicher Länge mit dem Unterschied, daß sie an eine Carboxylgruppe gebunden sind. Bevorzugt sind C₁-C₄-Alkanoyl bzw. -Alkansulfonyl.

Zur Salzbildung mit den freien Säuren (R⁴ = H) sind anorganische und organische Basen geeignet, wie sie dem Fachmann zur Bildung physiologisch verträglicher Salze bekannt sind. Beispielsweise seien genannt: Alkalihydroxide, wie Natrium- -oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroymethyl)-methylamin usw.
Bevorzugte Verbindungen der Formel I sind Verbindungen, in denen R¹ die Gruppe COOR⁴,
R³ Wasserstoff oder Hydroxyl,
R⁴ Wasserstoff oder C₁-C₆-Alkyl,
R⁵ Methansulfonyl,
X Sauerstoff oder CH₂,
W Wasserstoff oder Fluor bedeuten.

Die Erfindung betrifft ferner ein Verfahren zur Herstellung der Verbindungen der Formel IA. 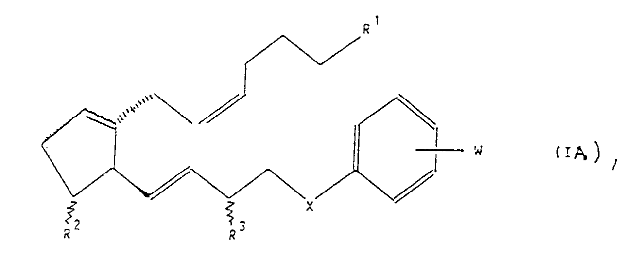
worin 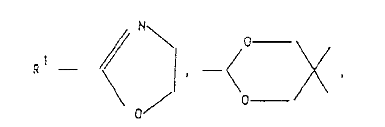
COOR⁴, wobei R⁴ Wasserstoff oder
oder einen gegebenenfalls durch Halogen, Phenyl, C₁-C₄-Alkoxy oder Di-(C₁-C₄)-alkylamino substituierten C₁-C₁₀-Alkyl-Rest, einen C₃-C₁₀-Cycloalkyl-, einen C₇-C₁₆-Aralkyl-, einen durch W substituierten Phenacyl-Rest, einen C₆-C₁₂-Aryl-Rest oder einen 5- oder 6-gliedrigen heterocyclischen Rest mit wenigstens einem N-, O- oder S-Atom bedeuten kann, oder R¹ ein CONHR⁵-Rest mit R⁵ in der Bedeutung Wasserstoff, C₁-C₁₀-Alkanoyl oder C₁-C₁₀-Alkansulfonyl sein kann,
R² und R³ jeweils ein Wasserstoffatom oder eine freie oder funktionell abgewandelte Hydroxygruppe, wobei die OH-Gruppe jeweils α- oder β-ständig sein kann, bedeuten,
X eine CH₂-Gruppe, ein O- oder S-Atom,
W Wasserstoff, -OR⁶ , Halogen, -CN-, -NO₂, Trifluormethyl oder COOR⁶,
R⁶ Wasserstoff, C₁-C₁₀-Alkyl, durch Halogen substituiertes C₆-C₁₂-Aryl oder C₇-C₁₆-Aralkyl sein kann und, falls R⁴ Wasserstoff bedeutet, deren Salze mit physiologisch verträglichen Basen, sowie die α-, β- oder γ-Cyclodextrinclathrate, sowie die mit Liposomen verkapselten Verbindungen der Formel IA bedeuten.
dadurch gekennzeichnet, daß man eine Verbindung der Formel II 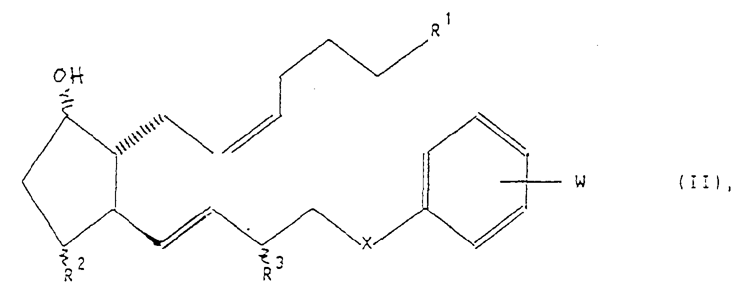
worin
R¹, R², R³ X und W die oben angegebenen Bedeutungen aufweisen und freie OH-Gruppen in R², R³ und W geschützt sind, mit Diethylaminoschwefeltrifluorid oder (HF)ₙ-Pyridin oder SeF₄-Pyridin umsetzt und geschützte Hydroxygruppen in R², R³ und W freisetzt und/oder freie Hydroxygruppen verestert, verethert und/oder eine veresterte Carboxygruppe verselft oder eine Carboxylgruppe mit einer physiologish verträglichen Base in ein Salz überführt oder mit α-, β- oder γ-Cyclodextrin zu einem Clathrat umsetzt oder mit Liposomen verkapselt.

Die Umsetzung der Verbindungen der allgemeinen Formel II zu den Verbindungen der allgemeinen Formel IA wird mit Diethylaminoschwefeltrifluorid bei -80°C bis +40°C, vorzugsweise bei -70°C bis +25°C durchgeführt. Als Lösungsmittel eignen sich Dichlormethan, 1.1.2-Trifluortrichlorethan, Pyridin, Toluol, Benzol, Ethylenchlorid u.a., vorzugsweise Toluol und Pyridin.
Die Freisetzung der funktionell abgewandelten Hydroxygruppen R², R³ und W erfolgt nach den dem Fachmann bekannten Methoden. Beispielsweise wird die Abspaltung von Etherschutzgruppen in einer wäßrigen Lösung einer organischen Säure, wie z.B. Essigsäure, Propionsäure, Zitronensäure u.a. oder in einer wäßrigen Lösung einer anorganischen Säure, wie z.B. Salzsäure, oder im Falle von Tetrahydropyranylethern unter Verwendung von Pyridinium-p-Toluolsulfonat, vorzugsweise in Alkoholen als Lösungsmittel oder unter Verwendung von wasserfreiem Magnesiumbromid, vorzugsweise in Diethylether als Lösungsmittel durchgeführt.

Zur Verbesserung der Löslichkeit wird bei Verwendung wäßrig-saurer Reaktionsbedingungen zweckmäßigerweise ein mit Wasser mischbares inertes Lösungsmittel zugesetzt. Als geeignet erweisen sich z.B. Alkohole wie Methanol und Ethanol, Ether wie Dimethoxyethan, Dioxan und Tetrahydrofuran, wobei Tetrahydrofuran bevorzugt angewendet wird.

Die Abspaltung der Silyletherschutzgruppen erfolgt beispielsweise mit Tetrabutylammoniumfluorid nach den dem Fachmann bekannten Methoden. Als Lösungsmittel sind beispielsweise Tetrahydrofuran, Diethylether, Dioxan, Methylenchlorid, usw. geeignet. Die Abspaltung wird vorzugsweise bei Temperaturen zwischen 20° und 80°C durchgeführt.

Die Verseifung der Acylgruppen und Prostaglandinester wird nach den dem Fachmann bekannten Methoden durchgeführt., wie beispielsweise mit basischen Katalysatoren wie z.B. mit Alkali- oder Erdalkali-carbonaten oder -hydroxiden in einem Alkohol oder der wäßrigen Lösung eines Alkohols. Als Alkohole kommen aliphatische Alkohole wie z.B. Methanol, Ethanol, Butanol usw. in Betracht, vorzugsweise jedoch Methanol. Als Alkalicarbonate und -hydroxide seien Lithium-, Natrium- und Kaliumsalze genannt. Bevorzugt sind die Lithium- und Kaliumsalze, Als Erdalkalicarbonate und -hydroxide eignen sich beispielsweise Calciumcarbonat, Calciumhydroxid und Bariumcarbonat. Die Umsetzung erfolgt allgemein bei -10° bis +70°C, vorzugsweise jedoch bei +25°C.

Die Einführung der Estergruppen CO₂R⁴ für R¹ bzw. CO₂R⁶ für W, bei welcher R⁴ bzw. R⁶ eine Alkylgruppe mit 1-10 C-Atomen darstellt, erfolgt nach den dem Fachmann bekannten Methoden. Die 1-Carboxyverbindungen (R⁴ = H bzw. R⁶ = H) werden beispielsweise mit Diazokohlenwasserstoffen in an sich bekannter Weise umgesetzt. Die Veresterung mit Diazokohlenwasserstoffen erfolgt z.B. dadurch, daß eine Lösung des Diazokohlenwasserstoffes in einem inerten Lösungsmittel, vorzugsweise in Diethylether, mit der 1-Carboxyverbindung, gelöst in dem gleichen oder in einem anderen inerten Lösungsmittel, wie z.B. Methylenchlorid, vermischt wird. Nach beendeter Umsetzung in 1 bis 60 Minuten wird das Lösungsmittel entfernt und der Ester in üblicher Weise gereinigt. Diazoalkane sind entweder bekannt oder können nach bekannten Methoden hergestellt werden (Org. Reactions Bd. 8, Seiten 389-394(1954)).

Die Einführung der Estergruppe CO₂R⁴ für R¹ bzw. CO₂R⁶ für W, bei welcher R⁴ bzw. R⁶ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base wie z.B. Pyridin, Dimethylaminopyridin, Triethylamin, in einem inerten Lösungsmittel wie z.B. Methylenchlorid, Ethylenchlorid, Chloroform, Essigsäureethylester, Tetrahydrofuran, vorzugsweise jedoch mit Chloroform umgesetzt. Die Reaktion wird bei Temperaturen zwischen -30°C und +50°C, vorzugsweise bei +10°C, durchgeführt.

Die Prostaglandinderivate der Formel I mit R⁴ bzw. R⁶ in der Bedeutung eines Wasserstoffatomes können mit geeigneten Mengen der entsprechenden anorganischen Basen unter Neutralisierung in Salze überführt werden. Beispielsweise erhält man beim Lösen der entsprechenden Prostaglandinsäuren in Wasser, welches stöchiometrische Mengen der Base enthält, nach Abdampfen des Wassers oder nach Zugabe eines mit Wasser mischbaren Lösungsmittels, z.B. Alkohol oder Aceton, das feste anorganische Salz.
Die Herstellung der Aminsalze erfolgt in üblicher Weise. Dazu löst man die Prostaglandinsäure in einem geeigneten Lösungsmittel, wie z.B. Ethanol, Aceton, Diethylether oder Benzol und setzt 1 bis 5 Äquivalente des jeweiligen Amins dieser Lösung zu. Dabei fällt das Salz gewöhnlich in fester Form an oder wird nach Verdampfen des Lösungsmittels in üblicher Weise isoliert.

Die funktionelle Abwandlung der freien Hydroxygruppen erfolgt nach den dem Fachmann bekannten Methoden. Zur Einführung der Esterschutzgruppen wird beispielsweise mit Dihydropyran oder Methylvinylether in Methylenchlorid oder Chloroform unter Verwendung katalytischer Mengen eines sauren Kondensationsmittels wie z.B. Toluolsulfonsäure, umgesetzt. Der jeweilige Enolether wird im Überschuß, vorzugsweise in der 1,2- bis 10-fachen Menge des theoretischen Bedarfs, zugesetzt. Die Umsetzung erfolgt normalerweise bei -10°C bis +30°C und ist nach 2 bis 45 Minuten beendet.

Zur Einführung von Silyletherschutzgruppen wird beispielsweise mit t-Butyl-diphenylchlorsilan oder t-Butyl-dimethylchlorsilan in Dimethylformamid unter Verwendung einer Base wie z.B. Imidazol, umgesetzt. Das jeweilige Silylchlorid wird im Überschuß, vorzugsweise in der 1,05- bis 4-fachen Menge des theoretischen Bedarfs, zugesetzt. Die Umsetzung erfolgt normalerweise bei 0°C bis 30°C und ist nach 1 bis 24 Stunden beendet.

Die Einführung der Acylschutzgruppen erfolgt, indem man eine Verbindung der Formel I in an sich bekannter Weise mit einem Carbonsäurederivat, wie z.B. Säurechlorid, Säureanhydrid usw., umsetzt.

Die neuen chemisch und metabolisch stabilen Δ⁸ -Prostaglandin-Derivate besitzen pharmakologische Eigenschaften, die denen des instabilen Thromboxan-A₂₋ (TXA₂) bzw. PGH₂ vergleichbar sind. Sie stellen als TXA₂/PGH₂-Rezeptoragonisten somit ein wertvolles diagnostisches Instrument zur Charakterisierung von Prostaglandinrezeptoren bzw. TXA₂/PGH₂-Rezeptorsubtypen, mit denen sich die Bedeutung der TXA₂/PGH₂-abhängigen Stimulation von Plättchen und Gefäßen nachweisen läßt, dar. Dies gilt sowohl für in vitro Tests wie z.B. Rezeptorcharakterisierung bzw. Verdrängung am Rezeptor, Thrombozytenaggregationshemmteste, Gefäßstreifenkonstriktion usw., als auch für pharmakologische Untersuchungen am Tier.

Die TXA₂/PGH₂-Rezeptoragonisten können zur spezifischen Abschwächung oder Aufhebung der Wirkung von Cyclooxygenasehemmstoffen, von TXA₂-Synthetasehemmstoffen sowie von TXA₂/PGH₂-Rezeptorblockern verwendet werden. Eine weitere Einsatzmöglichkeit besteht in der partiellen Herunterregulation der TXA₂/PGH₂-Wirkung bei Krankheitsbildern mit erhöhter Sensitivität gegenüber bzw. Produktion von Thromboxan wie z.B. die von Koronarien oder Gefäßen mit arteriosklerotischen Läsionen.

In Kombination mit einem TXA₂/PGH₂-Rezeptorantagonisten läßt sich der TXA₂/-PGH₂-Rezeptoragonist zur diagnostischen Abklärung der Beteiligung TXA₂/PGH₂-abhängiger Prozesse bei solchen Krankheitsbildern verwenden, die zu dieser Diagnostik keine systemische Gabe eines TXA₂/PGH₂-Rezeptoragonisten fordern, aber auch bei anderen Krankheitsbildern, sofern unerwünschten Wirkungen des TXA₂/PGH₂-Rezeptoragonisten durch einen Antagonisten gegengesteuert werden kann.

Die TXA₂/PGH₂-Rezeptoragonisten sind weiterhin geeignet zur lokalen Blutstillung bei Defekten der Plättchenfunktion, die auf einer Störung der TXA₂/PGH₂-Bildung und/oder Wirkung basieren.

Die Δ⁸- -Prostaglandin-Derivate dieser Erfindung können auch in Kombination, z.B. mit β-Blockern, Diuretika, Phosphodiesterasehemmern, Ca-Antagonisten oder nichtsteroidalen Entzündungshemmern, verwendet werden.

Die Dosis der Verbindungen ist 1-1000µg/kg/Tag, wenn sie am menschlichen Patienten verabreicht wird. Die Einheitsdosis für den pharmazeutischen akzeptablen Träger beträgt 10 µg bis 100 µg.

Für die parenterale Applikation werden sterile, injizierbare wäßrige oder ölige Lösungen benutzt. Für die orale Applikation sind beispielsweise Tabletten, Dragees oder Kapseln geeignet. Die Erfindung betrifft somit auch Arzneimittel auf Basis der Verbindungen der Formel I und üblicher Hilfs- und Trägerstoffe einschließlich Cyclodextrinclathrate und Verkapselung von Liposomen.

Die erfindungsgemäßen Wirkstoffe sollen in Verbindung mit den in der Galenik bekannten und üblichen Hilfsstoffen zum Beispiel zur Herstellung von Arzneimitteln dienen.

### Beispiel 1

(5Z,13E,15R)-15-Hydroxy-16-phenoxy-17,18,19,20-tetranor-5,8(9), 13-prostatriensäuremethylester

Die Lösung von 40 mg (66 µmol) der in Beispiel 1a dargestellten Verbindung löst man in 770 µl wasserfreiem Tetrahydrofuran, versetzt mit 151 µl einer 1M Tetrabutylammoniumfluoridlösung in Tetrahydrofuran und läßt drei Stunden unter einer Atmosphäre aus trockenem Argon rühren. Man gießt auf Eiswasser, extrahiert mit Diethylether, wäscht mit einer gesättigten Natriumchloridlösung nach und trocknet über Magnesiumsulfat. Das nach Lösungsmittelabzug im Wasserstrahlvakuum erhaltene Rohöl reinigt man durch Chromatographie an zwei analytischen Dünnschichtplatten. Als Laufmittel dient ein Gemisch aus n-Hexan und Ethylacetat, als Elutionsmittel Diethylether. Man isoliert 24 mg (65 µmol, 98%) der Titelverbindung als farbloses Öl.
- IR (Film):: 3600-3200, 3070, 3030, 3010, 2930, 2850, 1735, 1600, 1585, 1495, 1455, 1245, 1080, 1040, 970, 760 und 690 cm⁻¹.

### Beispiel 1a

(5Z,13E,15R)-15-t-Butyl-diphenylsilyloxy-16-phenoxy-17,18,19,20-tetranor-5,8-(9), 13-prostatriensäuremethyleester (A) und (5Z,9R,13E,15R)-9-Fluor-15-t-butyldiphenylsilyloxy-16-phenoxy-17,18,19,20-tetranor-5, 13-prostadiensäuremethylester (B)

125 mg (199 µmol) der in Beispiel 1b dargestellten Verbindung löst man in 3,6 ml wasserfreiem Toluol, versetzt mit 80 µl wasserfreiem Pyridin, kühlt unter einer Atmosphäre aus trockenem Argon auf -70°C, versetzt mit 60 µl Diethylaminoschwefeltrifluorid (DAST), läßt langsam auf -30°C erwärmen und rührt noch 2 Stunden. Man versetzt mit wenigen Tropfen einer gesättigten Natriumhydrogencarbonatlösung, läßt auf Raumtemperatur kommen, verdünnt mit Wasser und extrahiert mehrfach mit Dichlormethan. Man trocknet über Magnesiumsulfat und reinigt das nach Lösungsmittelabzug im Wasserstrahlvakuum erhaltene Rohöl durch Chromatographie an 7 analytischen Dünnschichtplatten. Als Laufmittel dient ein n-Hexan-Aceton-Gemisch, als Elutionsmittel Diethylether. Isoliert werden 40 mg (66 µmol, 33%) der Titelverbindung A sowie 47 mg (75 Mmol, 38%) der Titelverbindung B.
- IR (Film) von A:: 3070, 3040, 3000, 2940, 2850, 1735, 1600, 1590, 1490, 1450, 1430, 1360, 1300, 1245, 1170, 1110, 1045, 970, 820, 755, 740 und 705 cm⁻¹.
- IR (Film) von B:: 3070, 3010, 2950, 2860, 1735, 1600, 1585, 1495, 1450, 1425, 1360, 1245, 1110, 1045, 970, 820, 750, 740 und 705 cm⁻¹.

### Beispiel 1b

(5Z,9S,13E,15R)-9-Hydroxy-15-t-butyl-diphenylsilyloxy-16-phenoxy-17,18,19,20-tetranor-5,13-prostadiensäuremethylester

235 mg des in Beispiel 1c erhaltenen Rohproduktes löst man in 5 ml Dichlormethan, kühlt auf 0 bis 5°C und verestert mit einer etherischen Diazomethanlösung. Nach Abzug des Lösungsmittels chromatographiert man den Rückstand an ca. 30 ml feinem Kieselgel unter Druck. Als Laufmittel wird ein Gradientensystem aus n-Hexan und Ethylacetat verwendet. Isoliert werden 125 mg (199 µmol, 62% bezogen auf Edukt in Beispiel 1c).
- IR (Film ):: 3700-3300, 3070, 3040, 3000, 2950, 2930, 2890, 2860, 1735, 1600, 1585, 1495, 1450, 1430, 1245, 1110, 1040, 970, 820, 755, 740 und 705 cm⁻¹.

### Beispiel 1c

(5Z,9S,13E,15R)-9-Hydroxy-15-t-butyl-diphenylsilyloxy-16-phenoxy-17,18,19,20-tetranor-5,13-prostadiensäure

Die Lösung von 235 mg (321 µmol) der in Beispiel 1d dargestellten Verbindung in 1 ml Methanol versetzt man mit einer 5%igen Lithiumhydroxidlösung und rührt 5 Stunden bei 25°C. Man gießt in Eiswasser, stellt durch Zugabe einer gesättigten Citronensäurelösung einen pH-Wert von 4 bis 5 ein, extrahniert mehrfach mit Dichlormethan, wäscht mit Wasser und trocknet über Magnesiumsulfat. Nach Lösungsmittelabzug isoliert man 236 mg Rohprodukt, das ohne Reinigung weiter umgesetzt wird.

### Beispiel 1d

(5Z,9S,13E,15R)-9-Benzoyloxy-15-t-butyl-diphenylsilyloxy-16-phenoxy-17,18,19,20-tetranor-5,13-prostadiensäuremethylester

309 mg (343 µmol) der in Beispiel 1e dargestellten Verbindung löst man in 5 ml Dimethoxyethan, versetzt mit 519 mg Natriumjodid, 445 mg Zinkstaub, 300 µl Wasser und erhitzt 16 Stunden auf 80°C. Nach dem Erkalten wird filtriert, mit Dichlormethan nachgewaschen, mit Wasser extrahiert und die organische Phase über Magnesiumsulfat getrocknet. Nach Lösungsmittelabzug isoliert man 238 mg (325 µmol, 95%) der Titelverbindung als blassgelbes Öl.
- IR (Film):: 3070, 3030, 3000, 2950, 2950, 2930, 2860, 1735, 1715, 1600, 1585, 1490, 1450, 1270, 1245, 1110, 970, 820, 750, 740 und 705 cm⁻¹.

### Beispiel 1e

(5Z,9S,11R,13E,15R)-9-Benzoyloxy-1-(p-toluolsulfonyloxy)-15-t-butyl-diphenylsilyloxy-16-phenoxy-17,18,19,20-tetranor-5,13-prostadiensäuremethylester

300 mg (402 µmol) der in Beispiel 1f dargestellten Verbindung löst man in 2,7 ml wasserfreiem Pyridin, versetzt mit 354 mg p-Toluolsulfonsaurechlorid und erwärmt unter einer Atmosphäre aus trockenem Argon 7 Stunden auf 50°C. Man entfernt Pyridin durch wiederholte azeotrope Destillation mit Toluol, versetzt den Rückstand mit Wasser und extrahiert mehrfach mit Dichlormethan. Man wäscht mit gesättigter Natriumchloridlösung, trocknet über Magnesiumsulfat und reinigt den nach Lösungsmittelabzug im Wasserstrahlvakuum erhaltenen Rückstand durch Chromatographie an ca. 70 ml feinem Kieselgel unter Druck. Als Laufmittel dient ein Gradientensystem aus n-Hexan und Ethylacetat. Isoliert werden 533 mg (591 µmol, 92%) der Titelverbindung als farbloses Öl.
- IR (Film):: 3070, 3040, 3000, 297β, 2930, 2860, 1735, 1715, 1600, 1585, 1495, 1450, 1425, 1360, 1270, 1245, 1175, 1190, 1110, 965, 910, 855, 820, 755, 740, 710 und 665 cm⁻¹.

### Beispiel 1f

(5Z,9S,11R,13E,15R-9-Benzoyloxy-11-hydroxy-15-t-butyl-diphenylsilyloxy-16-phenoxy-17,18,19,20-tetranor-5,13-prostadiensäuremethylester

395 mg (475 µmol) der in Beispiel 19 dargestellten Verbindung setzt man in Analogie zu Beispiel 5c um und isoliert nach Aufarbeitung und Reinigung 327 mg (438 µmol, 92%) der Titelverbindung als farbloses Öl.
- IR (Film):: 3600-3200, 3070, 3040, 3010, 3000, 2950, 2930, 2860, 1735, 1720, 1600, 1585, 1495, 1450, 1430, 1275, 1245, 1175, 1115, 970, 825, 755, 740, 710, 615, 510 und 490 cm⁻¹.

### Beispiel 1g

(5Z,9S,11R,13E,15R)-9-Benzoyloxy-11-(tetrahydropyran-2-yloxy)-15-t-butyl-diphenyl-silyloxy-16-phenoxy-17,18,19,20-tetranor-5,13-prostadiensäuremethylester

3,05 g (5,15 mmol) des nach Beispiel 1h dargestellten polareren Alkohols löst man in 24 ml wasserfreiem Dimethylformamid, versetzt mit 713 mg Imidazol, 2,45 ml t-Butyldiphenylchlorsilan und rührt 16 Stunden bei 23°C unter einer Atmosphäre aus trockenem Argon. Man gießt auf Eiswasser, extrahiert mehrfach mit Diethylether, wäscht die vereinigten organischen Extrakte mit Wasser und gesättigter Natriumchloridlösung, trocknet über Magnesiumsulfat und reinigt den nach Filtration und Lösungsmittelabzug erhaltenen Rückstand durch Chromatographie an ca. 250 ml feinem Kieselgel mittels eines Gradientensystems aus n-Hexan und Ethylacetat. Man isoliert 3,59 g (4,32 mmol, 84%) der Titelverbindung als farbloses Öl.
- IR (Film):: 3070, 3040, 3010, 2940, 2860, 1735, 1715, 1600, 1585, 1495, 1450, 1425, 1270, 1245, 1110, 1025, 970, 865, 820, 755, 710 und 705 cm⁻¹.

### Beispiel 1h

(5Z,9S,11R,13E,15R)-9-Benzoyloxy-11-(tetrahydropyran-2-yloxy)-15-hydruxy-16 phenoxy-17,18,19,20-tetranor-5,13-prostadiensäuremethylester (A) und (5Z,9S, 11R,13E,15S)-9-Benzoyloxy-11-(tetrahydropyran-2-yloxy)-15-hydroxy-16-phenoxy-17,18,19,20-tetranor-5,13-prostadiensäuremethylester (B)

7,49 g (12,7 mmol) des in Beispiel 1i dargestellten Ketons reduziert man in Analogie zu Beispiel 9d und isoliert nach Aufarbeitung und chromatographischer Trennung 3,43 g (5,79 mmol, 46%) der Titelverbindung 8 als unpolarere, sowie 3,71 g (6,25 mmol, 49%) der Titelverbindung A als polarere Komponente.
- IR (Film) von A und B:: 3600-3200, 3060, 3010, 2949, 2870, 1735, 1710, 1600, 1495, 1450, 1360, 1275, 1245, 1115, 1070, 1025, 970, 865, 810, 755, 715 und 690 cm⁻¹.

### Beispiel 1i

(5Z,9S,11R,13E,15R)-9-Benzoyloxy-11-(tetrahydropyran-2-yloxy)-15-oxo-16-phenoxy-17,18,19,20-tetranor-5,13-prostadiensäuremethylester

Die Lösung von 7,70 g (16,8 mmol) (1R,2R,3R,5S)-7-[2-Formyl-3-(tetrahydropyran-2-yloxy)-5-benzoyloxycyclopentyl]-5-(Z)-heptensäuremethylester löst man in 210 ml wasserfreiem Dimethoxyethan, versetzt mit 17,8 g Dimethyl-(2-oxo-3-phenoxy-propyl)-phosphonat-Lithiumsalz und rührt 2 Tage bei 23°C unter einer Atmosphäre aus trockenem Argon. Man gießt auf Eiswasser, extrahiert mehrfach mit Diethylether, wäscht die vereinigten organischen Extrakte mit Wasser und gesättigter Natriumchloridlösung, trocknet über Magnesiumsulfat und reinigt den nach Filtration und Lösungsmittelabzug erhaltenen Rückstand durch Chromatogra- phie an ca. 600 ml feinem Kieselgel mittels eines Gradientensystems aus n-Hexan und Ethylacetat. Isoliert wurden neben 1,59 g Ausgangsmaterial 7,49 g (12,7 mmol, 75%) der Titelverbindung als farbloses Öl.
- IR (Film):: 3070, 3010, 2950, 2870, 1735, 1715, 1695, 1620, 1600, 1495, 1450, 1275, 1120, 1030, 975, 870, 815, 760, 720 und 695 cm⁻¹.

### Beispiel 2

(5Z,13E,15R)-15-Hydroxy-16-phenoxy-17,18,19,20-tetranor-5,8(9), 13-prostatiensäure

24 mg (65 µmol) der in Beispiel 1 dargestellten Verbindung löst man in 940 µl Methanol, versetzt mit 315 µl einer 8%igen Kaliumhydroxidlösung und rührt 5 Stunden bei 25°C. Die Aufarbeitung erfolgt in Analogie zu Beispiel 1c. Isoliert werden 21 mg (59 µmol, 91%) der Titelverbindung als farbloses Öl.
- IR (Film):: 3600-2400, 30070, 3040, 3010, 2930, 2850, 1710, 1600, 1585, 1495, 1455, 1245, 1080, 1040, 970, 755 und 690 cm⁻¹.

### Beispiel 3

(5Z,13E,15S)-15-Hydroxy-16-phenoxy-17,18,19,20-tetranor-5,8(9),13-prostatrien-säuremethylester

42 mg (69 µmol) der in Beispiel 3a dargestellten Verbindung setzt man in Analogie zu Beispiel 1 um und isoliert nach Aufarbeitung und Reinigung 23 mg (62 µmol, 90%) der Titelverbindung als farbloses Öl.
- IR (Film):: 3600-3200, 3080, 3040, 3010, 2930, 2850, 1740, 1600, 1590, 1500, 1455, 1245, 1080, 1040, 970, 755 und 690 cm⁻¹.

### Beispiel 3a

(5Z,13E,15S)-15-t-Butyl-diphenylsilyloxy-16-phenoxy-17,18,19,20-tetranor-5,8 (9), 13-prostatriensäuremethylester (A) und (5Z,9R,13E,15S)-9-Fluor-15-t-butyldiphenylsilyloxy-16-phenoxy-17,18,19,20-tetranor-5,13-prostadiensäuremethyl- ester (B)

103 mg (164 µmol) der in Beispiel 3b dargestellten Verbindung setzt man in Analogie zu Beispiel 1a um und isoliert nach Aufarbeitung und Reinigung 42 mg (69µmol, 42%) der Titelverbindung A sowie 41 mg (65 µmol, 40%) der Titelverbindung B.
- IR (Film) von A:: 3070, 3040, 3010, 2950, 2930, 2850, 1735, 1600, 1585, 1490, 1425, 1360, 1300, 1240, 1170, 1110, 1040, 970, 820, 750, 740 und 705 cm⁻¹.
- IR (Film) von B:: 3070, 3050, 3010, 2950, 2930, 2860, 1735, 1600, 1590, 1495, 1430, 1360, 1245, 1170, 1110, 1045, 970, 820, 755, 740 und 705 cm⁻¹.

### Beispiel 3b

(5Z,9S,13E,15S)-9-Hydroxy-15-t-butyl-diphenylsilyloxy-16-phenoxy-17,18,19,20-tetranor-5,13-prostadiensäuremethylester

420 mg des in Beispiel 3c dargestellten Rohproduktes setzt man in Analogie zu Beispiel 1b um und isoliert nach Aufarbeitung und Reinigung 233 mg (372 µmol, 67% bezogen auf Edukt in Beispiel 3c) der Titelverbindung als farbloses Öl.
- IR (Film):: 3600-3300, 3070, 3040, 3000, 2950, 2950, 2930, 2860, 1735, 1600, 1590, 1495, 1425, 1240, 1110, 1040, 970, 820, 755, 740 und 705 cm⁻¹.

### Beispiel 3c

(5Z,9S,13E,15S)-9-Hydroxy-15-t-butyl-diphenylsilyloxy-16-phenoxy-17,18,19,20-tetranor-5,13-prostadiensäure

406 mg (555 µmol) der in Beispiel 3d dargestellten Verbindung setzt man in Analogie zu Beispiel 1c um und isoliert nach Aufarbeitung 423 mg Rohprodukt, das ohne Reinigung weiter umgesetzt wird.

### Beispiel 3d

(5Z,9S,13E,15S)-9-Benzoyloxy-15-t-butyl-diphenylsilyloxy-16-phenoxy-17,18,19,20-tetranor-5,13-prostadiensäuremethylester

533 mg (591 µmol) der in Beispiel 3e dargestellten Verbindung setzt man in Analogie zu Beispiel 1d um und isoliert nach Aufarbeitung und Reinigung 421 mg (576 µmol, 97%) der Titelverbindung als blassgelbes Öl.
- IR (Film):: 3070, 3040, 3000, 2950, 2930, 2860, 1735, 1710, 1600, 1585, 1490, 1450, 1430, 1270, 1245, 1110, 970, 820, 750, 740 und 705 cm⁻¹.

### Beispiel 3e

(5Z,9S,11R,13E,15S)-9-Benzoyloxy-11-(p-toluolsulfonyloxy)-15-t-butyl-diphenylsilyloxy-16-phenoxy-17,18,19,20-tetranor-5,13-prostadiensäuremethylester

479 mg (641 µmol) der in Beispiel 3f dargestellten Verbindung setzt man in Analogie zu Beispiel 1e um und isoliert nach Aufarbeitung und Reinigung 533 mg (591 µmol, 92%) der Titelverbindung als farbloses Öl.
- IR (Film):: 3070, 3040, 2940, 2860, 1720 (breit), 1600, 1495, 1450, 1430, 1360, 1270, 1245, 1175, 1110, 970, 910, 850, 820, 750, 710 und 665 cm⁻¹.

### Beispiel 3f

(5Z,9S,11R,13E,15S)-9-Benzoyloxy-11-hydroxy-15-t-butyl-diphenylsilyloxy-16-phenoxy-17,18,19, 20-tetranor-5,13-prostadiensäure-methylester

960 mg (1,16 mmol) der in Beispiel 3g dargestellten Verbindung setzt man in Analogie zu Beispiel 5c um und isoliert nach Aufarbeitung und Reinigung 607 mg (813 µmol, 70%) der Titelverbindung als farbloses Öl.
- IR (Film):: 3600-3200, 3070, 3040, 3010, 3000, 2950, 2930, 2860, 1735, 1715, 1600, 1585, 1495, 1450, 1425, 1360, 1315, 1275, 1245, 1175, 1115, 970, 825, 755, 740, 705, 690, 615, 510 und 490 cm⁻¹.

### Beispiel 3g

(5Z,9S,11R,13E,15S)-9-Benzoyloxy-11-(tetrahydropyran-2-yloxy)-15-t-butyl-diphenylsilyloxy-16-phenoxy-17,18,19,20-tetranor-5,13-prostadiensäure-methylester

2,77 g (4,67 mmol) der in Beispiel 1h dargestellten unpolareren Alkohols syliert man in Analogie zu Beispiel 1g um und isoliert nach Aufarbeitung und Reinigung 3,31 g (3,98 mmol, 85%) der Titelverbindung als farbloses Öl.
- IR (Film):: 3070, 3050, 3010, 2940, 2860, 1735, 1715, 1600, 1585, 1450, 1430, 1270, 1245, 1110, 1025, 970, 860, 820, 740 und 705 cm⁻¹.

### Beispiel 4

(5Z,13E,15S)-15-Hydroxy-16-phenoxy-17,18,19,20-tetranor-5,8(9),13-prostatriensäure

23 mg (62 µmol) der in Beispiel 3 dargestellten Verbindung setzt man in Analogie zu Beispiel 2 um und isoliert nach Aufarbeitung 19,8 mg (56 µmol, 89%) der Titelverbindung als farbloses Öl.
- IR (Film):: 3600-2400, 3070, 3040, 3010, 2930, 2850, 1710, 1600, 1590, 1500, 1460, 1245, 1080, 1040, 970, 755 und 690 cm⁻¹.

### Beispiel 5

(5Z,11S,13E,15S)-11,15-Dihydroxy-16-phenoxy-17,18,19,20-tetranor-5,8(9),13-prostatriensäure

20 mg (41 µmol) der in Beispiel 5a dargestellten Verbindung verseift man in Analogie zu Beispiel 1c um und isoliert nach Aufarbeitung und Reinigung 14 mg (38 µmol, 92%) der Titelverbindung als farbloses Öl.
- IR (Film):: 3600-2500, 3050, 3010. 2930. 2870. 1710, 1600, 1585, 1495, 1245, 1080, 1040, 975, 810, 755 und 690 cm⁻¹.

### Beispiel 5a

(5Z,11S,13E,15R)-11-Benzoyloxy-15-hydroxy-16-phenoxy-17,18,19,20-tetranor-5,8(9),13-prostatriensäuremethylester

33 mg (45 µmol) der in Beispiel 5b dargestellten Verbindung setzt man in Analogie zu Beispiel 1 um und isoliert nach Aufarbeitung und Reinigung 20 mg (41 µmol, 90%) der Titelverbindung als farbloses Öl.
- IR (Film):: 3600-3200, 3060, 3010, 2950, 2870, 1735, 1710, 1600, 1495, 1450, 1360, 1245, 1110, 1070, 1025, 970, 755, 715 und 695 cm⁻¹.

### Beispiel 5b

(5Z,11S,13E,15R)-11-Benzoyloxy-15-t-butyl-diphenylsilyloxy-16-phenoxy-17,18,19, 20-tetranor-5,8(9),13-prostatriensäuremethylester

38 mg (61 µmol) der in Beispiel 5c dargestellten Verbindung löst man in 1,5 ml wasserfreiem Toluol, versetzt mit 35 mg Triphenylphosphin, 16,4 mg Benzoesäure mit 21 µl Azodicarbonsäuressäure-diethylester (DEAD). Man rührt 3 Stunden bei 25°C unter einer Atmosphäre aus trockenem Argon, versetzt mit Wasser, extrahiert mehrfach mit Diethylether, trocknet über Magnesiumsulfat und reinigt den nach Lösungsmittelabzug erhaltenen Rückstand durch Chromatographie an 7 analytischen Dünnschichtplatten. Als Laufmittel dient ein Gemisch aus Ethylacetat und n-Hexan, als Elutionsmittel Ethylacetat. Isoliert werden 33 mg (45 µmol, 74%) der Titelverbindung als farbloses Öl.
- IR (Film):: 3070, 3030, 3000, 2950, 2930, 2860, 1735, 1715, 1600, 1590, 1490, 1490, 1450, 1425, 1270, 1245, 1110, 970, 820, 750 und 705 cm⁻¹.

### Beispiel 5c

(5Z,11R,13E,15R)-11-Hydroxy-15-t-butyl-diphenylsilyloxy-16-phenoxy-17,18,19,20- tetranor-5,8(9),13-prostadiensäuremethylester

69 mg (97 µmol) der in Beispiel 5d dargestellten Verbindung löst man in 1,5 ml Methanol, versetzt mit 8 mg Pyridinium-p-Toluolsulfonat (PPTs) und erwärmt unter einer Atmosphäre aus trockenem Argon 2 Stunden auf 55°C. Nach dem Erkalten versetzt man mit Dichlormethan, wäscht mit Wasser und gesättigter Natriumchloridlösung, trocknet über Magnesiumsulfat und reinigt den nach Lösungsmittelabzug erhaltenen Rückstand durch Chromatographie an 3 analytischen Dunnschichtplatten. Als Laufmittel dient ein Gemisch aus Ethylacetat und n-Hexan, als Elutionsmittel Ethylacetat. Isoliert werden 38 mg (61 µmol, 62%) der Titelverbindung als farbloses Öl.
- IR (Film):: 3600-3200, 3070, 3030, 3000, 2940, 2860, 1735, 1600, 1585, 1495, 1450,1245, 1110, 1040, 970, 815, 755, 740 und 705 cm⁻¹.

### Beispiel 5d

(5Z,11R,13E,15R)-11-(Tetrahydropyran-2-yloxy)-15-t-butyl-diphenylsilyloxy-16-phenoxy-17,18,19,20-tetranor-5,8(9),13-prostadiensäuremethylester (A) und (5Z,9R,11R,13E,15R)-9-Fluor-11-(tetrahydropyran-2-yloxy)-15-t-butyl-diphenyl- silyloxy-16-phenoxy-17,18,19,20-tetranor-5,8(9),13-prostadiensäuremethylester (B)

213 mg (293 µmol) der in Beispiel 5e dargestellten Verbindung setzt man in Analogie zu Beispiel 1a um und isoliert nach Aufarbeitung und Reinigung 80 mg (113 µmol, 39%) der Titelverbindung A sowie 91 mg (125 µmol, 43%) der Titelverbindung B.
- IR (Film) von A:: 3070, 3040, 3010, 2940, 2860, 1735, 1600, 1595, 1490, 1450, 1425, 1355, 1245, 1110, 1045, 970, 870, 820, 755, 740 und 705 cm⁻¹.
- IR (Film) von B:: 3060, 3030, 3000, 2950, 2860, 1735, 1600, 1590, 1495, 1450, 1425, 1360, 1245, 1110, 1045, 970, 865, 820, 750, 750 und 705 cm⁻¹.

### Beispiel 5e

(5Z,9S,11R,13E,15R)-9-Hydroxy-11-(tetrahydropyran-2-yloxy)-15-t-butyldiphenylsilyloxy-16-phenoxy-17,18,19,20-tetranor-5,13-prostadiensäuremethylester

1,49 g (1,79 mmol) der nach Beispiel 1g dargestellten Verbindung setzt man in Analogie zu Beispiel 1c und 1b um und isoliert nach Aufarbeitung und Reinigung 1,14 g (1,57 mmol, 88%) der Titelverbindung als farbloses Öl.
- IR (Film):: 3600-3200, 3070, 3040, 3000, 2960, 2940, 2860, 1735, 1600, 1585, 1495, 1245, 1110, 1040, 970, 865, 820, 755, 740 und 705 cm⁻¹.

### Beispiel 6

(5Z,11S,13E,15R)-11,15-Dihydroxy-16-phenoxy-17,18,19,20-tetranor-5,8(9)-13-prostatriensäuremethylester

8,7 mg (18 µmol) der in Beispiel 5 dargestellten Verbindung verestert man in Analogie zu Beispiel 1b und isoliert nach Lösungsmittelabzug 6,5 mg (17 µmol), 93%) der Titelverbindung als farbloses Öl.
- IR (Film):: 3600-3200, 3080, 3010, 2940, 2870, 1735, 1600, 1590, 1495, 1245, 1080, 1040, 970, 810, 755 und 690 cm⁻¹.

### Beispiel 7

(5Z,11S,13E,15S)-11,15-Dihydroxy-16-phenoxy-17,18,19,20-tetranor-5,8(9),13-prostatriensäure

109 mg (222 µmol) der in Beispiel 7a dargestellten Verbindung verseift man in Analogie zu Beispiel 1c und isoliert nach Aufarbeitung und chromatographisher Reinigung 65 mg (175 µmol, 79%) der Titelverbindung als farbloses Öl.
- IR (Film):: 3600-2500, 3070, 3050, 3020, 2940, 2870, 1710, 1600, 1590, 1500, 1245, 1080, 755 und 695 cm⁻¹.

### Beispiel 7a

(5Z,11S,13E,15S)-11-Benzoyloxy-15-hydroxy-16-phenoxy-17,18,19,20-tetranor-5,8-(9),13-prostatriensäuremethylester (A) und
(5Z,9R,115,13E,15S)-9-Fluor-11-benzoyloxy-15-hydroxy-16-phenoxy -17,18,19,20-tetranor-5,13-prostadiensäuremethylester (B)

355 mg (ca. 500 µmol) des in Beispiel 7b dargestellten Gemisches setzt man in Analogie zu Beispiel 1 um und isoliert nach Aufarbeitung und chromatographischer Reinigung 109 mg (222 µmol, 36% bezogen auf Edukt in Beispiel 7d)) der Titelverbindung A sowie 139 mg (273 µmol, 45% bezogen auf Edukt in Beispiel 7d) die Titelverbindung B.
- IR (Film) von A und B:: 3600-3200, 3050, 3010, 2950, 2870, 1735, 1710, 1600, 1495, 1450, 1245, 1110, 1065, 970, 755, 715 und 695 cm⁻¹.

### Beispiel 7b

(5Z,11s,13E,15S)-11-Benzoyloxy-15-t-butyl-diphenylsilyloxy-16-phenoxy-17,18,19, 20-tetranor-5,8(9),13-prostatriensäuremethylester und
(5Z,9R,11S,13E,15S)-9-Fluor-11-benzoyloxy-15-t-butyl-diphenylsilyloxy-16-phenoxy-17,18,19,20-tetranor-5,13-prostadiensäuremethylester

350 mg (ca. 550 µmol) des in Beispiel 3c dargestellten Gemisches setzt man in Analogie zu Beispiel 5b um und isoliert nach Aufarbeitung und Reinigung 359 mg (ca. 500 µmol, 91%) eines Gemisches der beiden Titelverbindungen, das ohne Trennung weiter umgesetzt wird.
- IR (Film):: 3070, 3050, 3010, 2950, 2860, 1735, 1710, 1600, 1595, 1495, 1425, 1360, 1245, 1170, 1100, 1040, 970, 820, 755, 740 und 705 cm⁻¹.

### Beispiel 7c

(5Z,11R,13E,15S)-11-Hydroxy-19-t-butyl-diphenylsilyloxy-16-phenoxy-17,18,19,20-tetranor-5,8(9),13-prostatriensäuremethylester und
(5Z,9R,11R,13E,15S)-9-Fluor-11-hydroxy-15-t-butyl-diphenylsilyloxy-16-phenoxy-17,18,19,20-tetranor-5,13-prostadiensäuremethylester

389 mg (ca. 550 µmol) des in Beispiel 7d dargestellten Gemisches setzt man in Analogie zu Beispiel 5c um und isoliert nach Aufarbeitung und Reinigung 350 mg (ca. 550 µmol, ca. 100%) eines Gemisches der beiden Titelverbindungen, das ohne Trennung weiter umgesetzt wird.
- IR (Film):: 3600-3200, 3070, 3030, 3010, 2950, 2870, 1735, 1600. 1590, 1495, 1450, 1245, 1110, 1035, 970, 820, 755, 740 und 705 cm⁻¹.

### Beispiel 7d

(5Z,11R,13E,15S)-11-(Tetrahydropyran-2-yloxy)-15-t-butyl-diphenylsilyloxy-16-phenoxy-17,18,19,20-tetranor-5,8(9),13-prostatriensäuremethylester und
(5Z,9R,11R,13E,15S)-9-Fluor-11-(tetrahydropyran-2-yloxy)-15-t-butyl-diphenylsilyloxy-16-phenoxy-17,18,19,20-tetranor-5,13-prostadiensäuremethylester

434 mg (609 µmol) der in Beispiel 7e dargestellten Verbindung setzt man in Analogie zu Beispiel 1a um und isoliert nach Aufarbeitung und Reinigung 389 mg (ca. 540 µmol, ca. 88%) eines Gemisches der beiden Titelverbindungen, das ohne Trennung weiter umgesetzt wird.
- IR (Film):: 3080, 3040, 3010, 2950, 2860, 1730, 1600, 1595, 1495, 1450, 1425, 1355, 1245, 1110, 1045, 970, 865, 820, 755, 740 und 705 cm⁻¹.

### Beispiel 7e

(5Z,95,11R,13E,15S)-9-Hydroxy-11-(tetrahydropyran-2-yloxy)-15-t-butyl-diphenylsilyloxy-16-phenoxy-17,18,19,20-tetranor-5,13-prostadiensäuremethylester

755 mg (908 µmol) der nach Beispiel 3g dargestellten Verbindung setzt man in Analogie zu den Beispielen 1c und 1b um und isoliert nach Aufarbeitung und Reinigung 523 mg (719 µmol, 79%) der Titelverbindung als farbloses Öl.
- IR (Film):: 3600-3200, 3070, 3040, 3010, 2960, 2850, 1735, 1600, 1585, 1495, 1245, 1110, 1040, 970, 870, 820, 755, 740 und 705 cm⁻¹.

### Beispiel 8

(5Z,11S,13E,15S)-11,15-Dihydroxy-16-phenoxy-17,18,19,20-tetranor-5,8(9),13-prostatriensäuremethylester

11 mg (30 µmol) der in Beispiel 7 dargestellten Verbindung verestert man in Analogie zu Beispiel 1b und isoliert nach Aufarbeitung und Reinigung 10 mg (26 µmol, 88%) der Titelverbindung als farbloses Öl.
- IR (Film):: 3600-3300, 3070, 3050, 3010, 2940, 2870, 1740, 1600, 1590, 1500, 1245, 1080, 1040, 755 und 695 cm⁻¹.

### Beispiel 9

(5Z,11R,13E,15R)-11,15-Dihydroxy-17-phenoxy-17,18,19,20-trinor-5,8(9),13-prostatriensäuremethylester

316 mg (572 µmol) der in Beispiel 9a dargestellten Verbindung A versetzt man mit 14 ml eines Eisessig:Wasser:Tetrahydrofuran (65:35:10)-Gemisches und läßt 16 Stunden bei 23°C rühren. Man engt im Wasserstrahlvakuum ein und entfernt restliche Essigsäure azeotrop durch wiederholte Zugabe von Toluol. Man isoliert 214 mg (556 µmol, 97%) der Titelverbindung als farbloses Öl, das man ohne Reinigung weiter umsetzt.
- IR (Film):: 3600-3200, 3070, 3060, 3020, 2940, 2870, 1735, 1600, 1495, 1450, 1410, 1245. 1035, 970, 750 und 700 cm⁻¹.

### Beispiel 9a

(5Z,11R,13E,15R)-11,15-Bis-(tetrahydropyran-2-yloxy)-17-phenyl-18,19,20-trinor-5,8(9),13-prostatriensäuremethylester (A) und
(5Z,9R,11R,13E,15R)-9-Fluor-11,15-bis-(tetrahydropyran-2-yloxy)-17-phenyl-18, 19,20-trinor-5,13-prostadiensäuremethylester (B)

1,24 g (2,18 mmol) der in Beispiel 9b dargestellten Verbindung setzt man in Analogie zu Beispiel 1a um und isoliert nach Aufarbeitung und Reinigung 316 mg (572 µmol, 26%) der Titelverbindung A sowie 360 mg (629 µmol, 29%) der Titelverbindung (B).
- IR (Film) von A:: 3090, 3060, 3030, 2950, 2870, 1740, 1605, 1455, 1440, 1200, 1200, 1135, 1120, 1080, 1030, 980, 870, 815, 750 und 700 cm⁻¹.
- IR (Film) von B:: 3090, 3070, 3030, 3010, 2940, 2870, 1740, 1455, 1440, 1355, 1075, 1030, 975, 870, 815, 750 und 7080 cm⁻¹.

### Beispiel 9b

(5Z,9S,11R,13E,15R)-9-Hydroxy-11,15-bis(tetrahydropyran-2-yloxy)-17-phenyl-18, 19,20-trinor-5,13-prostadiensäuremethylester

1,51 g (2,24 mmol) der in Beispiel 9c dargestellten Verbindung löst man in 50 ml wasserfreiem Methanol, versetzt mit 0,66 g fein pulverisiertem Kaliumcarbonat und rührt 60 Stunden bei 23°C unter einer Atmosphäre aus trockenem Argon. Man verdünnt mit Wasser, extrahiert mehrfach mit Diethylether, wäscht die vereinigten organischen Extrakte mit Wasser und gesättigter Natriumchloridlosung, trocknet über Magnesiumsulfat und reinigt den nach Lösungsmittelabzug erhaltenen Rückstand durch Chromatographie an ca. 100 ml feinem Kieselgel mit einem Laufmittelgemisch aus n-Hexan und Ethylacetat. Isoliert werden 1,24 g (2,18 mmol, 91%) der Titelverbindung als farbloses Öl.
- IR (Film):: 3600-3200, 3090, 3060, 3030, 3010, 2950, 2870, 1740, 1600, 1455, 2440, 1250, 1135, 1080, 1025,980, 870, 815, 750 und 705 cm⁻¹.

### Beispiel 9c

(5Z,9S,11R,13E,15R)-9-Benzoyloxy-11,15-bis-(trahydropyran-2-yloxy)-17-phenyl-18,19,20-trinor-5,13-prostadiensäuremethylester

Die Lösung von 1,41 g (2,3 mmol) der in Beispiel 9d dargestellten Verbindung B in 50 ml wasserfreiem Dichlormethan versetzt man mit 6 mg p-Toluolsulfonsaure und 260 µl Dihydropyran. Man läßt 1 Stunde bei 23°C unter einer Atmosphäre aus trockenem Argon rühren, versetzt die violett gefärbte Lösung mit 25 ml einer 10%igen Natriumhydrogencarbonatlösung, trennt die organische Phase ab, wäscht mehrfach mit Wasser nach und trocknet über Magnesiumsulfat. Nach Filtration und Abzug des Lösungsmittels im Wasserstrahlvakuum isoliert man 1,77 g eines gelben Öles, das an ca. 100 ml feinem Kieselgel mittels eines Gemisches aus n-Hexan und Ethylacetat chromatographisch gereinigt wird. Man isoliert 1,51 g (2,24 mmol, 97%) der Titelverbindung als farbloses Öl.
- IR (Film):: 3090, 3060, 3030, 3010, 2940, 2870, 1740, 1600, 1585, 1450, 1440, 1350, 1275, 1130, 1115, 1025, 975, 870, 815, 750 und 700 cm⁻¹.

### Beispiel 9d

(5Z,9S,11R,13E,15S)-9-Benzoyloxy-11-(tetrahydropyran-2-yloxy)-15-hydroxy-17-phenyl-18,19,20-trinor-5,13-prostadiensäuremethylester (A) und
(5Z,9S,11R,13E,15R)-9-Benzoyloxy-11-(tetrahydropyran-2-yloxy)-15-hydroxy-17-phenyl-18,19,20-trinor-5,13-prostadiensäuremethylester (B)

3,43 g (5,882 mmol) des in Beispiel 9e dargestellten ungesättigten Ketons löst man in 68 ml Methanol, kühlt unter einer Atmosphäre aus trockenem Argon auf -40°C und versetzt mit 1,39 g Natriumborhydrid. Nach einer Stunde quencht man durch Zugabe von 3 ml Eisessig, läßt auf Raumtemperatur erwärmen und extrahiert mehrfach mit Diethylether. Man trocknet über Magnesiumsulfat und reinigt den nach Lösungsmittelabzug erhaltenen Rückstand durch Chromatographie an ca. 150 g feinem Kieselgel. Als Elutionsmittel dient ein Gemisch aus n-Hexan und Ethylacetat. Isoliert werden 1,41 g (2,3 mmol, 41%) einer unpolaren Komponente, der man die Struktur B zuordnet neben 1,11 g (1,88 mmol, 32%) einer polareren Komponente, der die Struktur A zugeordnet wird.
- IR (Film) von A:: 3600-3200, 3090, 3070, 3030, 3010, 2950, 2870, 1735, 1720, 1600, 1585, 1445, 1440, 1355, 1315, 1275, 1120, 1075, 1030, 975, 870, 810, 750, 720 und 705 cm⁻¹.
- IR (Film) von B:: 3600-3200, 3090, 3070, 3030, 3010, 2950, 2860, 1735, 1720, 1600, 1580, 1440, 1435, 1315, 1275, 1120, 1075, 1030, 975, 870, 815, 750, 715 und 705 cm⁻¹.

### Beispiel 9e

(5Z,9S,11R,13E)-9-Benzoyloxy-11-(tetrahydropyran-2-yloxy)-15-oxo-17-phenyl-18,19,20-trinor-5,13-prostadiensäuremethylester

Zu der Mischung aus 85 mg wasserfreiem Lithiumchlorid in 20 ml wasserfreiem Acetonitril gibt man unter einer Atmosphäre aus trockenem Argon die Lösung von 513 mg Dimethyl-(2-oxo-4-phenyl-butyl)-phosphonat in 3 ml Acetonitril, versetzt mit 140 µl DBU und sodann mit der Lösung von 458 mg (1,00 mmol) (1R,2R,3R,5S)-7-[2-Formyl-3-(tetrahydropyran-2-yloxy)-5-benzoyloxycyclopentyl]-5(Z)-heptensäuremethylester in 4 ml Acetonitril und läßt 17 Stunden bei 23°C rühren. Man verdünnt mit Methyl-t-butylether, filtriert, wäscht das Filtrat mit Wasser und gesättigter Natriumchloridlösung und trocknet über Magnesiumsulfat. Das nach Lösungsmittelabzug erhaltene Rohprodukt reinigt man durch Chromatographie an ca. 50 ml feinem Kieselgel unter Verwendung eines Gemisches aus n-Hexan und Ethylacetat. Isoliert werden 567 mg (963 µmol, 96%) der Titelverbindung als farbloses Öl.
- IR (Film):: 3090, 3060, 3030, 3010, 2950, 2870, 1735, 1720, 1675, 1630, 1605, und 700 cm⁻¹.

### Beispiel 10

(5Z,11R,13E,15R)-11,15-Dihydroxy-17-phenyl-18,19,20-trinor-5,8(9),13-prostatriensäure

214 mg (556 µmol) der in Beispiel 9 dargestellten Verbindung setzt man in Analogie zu Beispiel 2 um und isoliert nach Aufarbeitung 95 mg (256 µmol, 40%) der Titelverbindung als farbloses Öl.
- IR (Film):: 3600-2400, 3080, 3060, 3020, 2930, 2880, 1710, 1600, 1495, 1450, 1410, 1240, 1030, 970, 750 und 700 cm⁻¹.

### Beispiel 11

(5Z,11R,13E,15S)-11,15-Dihydroxy-17-phenyl-18,19,20-trinor-5,8(9),13-prostatriensäuremethylester

247 mg (447 µmol) der in Beispiel 11a dargestellten Verbindung A setzt man in Analogie zu Beispiel 9 um und isoliert nach Aufarbeitung und Reinigung 165 mg (428 µmol, 96%) der Titelverbindung als farbloses Öl.
- IR (Film):: 3600-3200, 3080, 3060, 3030, 2940, 2860, 1740, 1605, 1500, 1455, 1245, 1050, 970, 750 und 695 cm⁻¹.

### Beispiel 11a

(5Z,11R,13E,15S)-11,15-Bis-(tetrahydropyran-2-yloxy)-17-phenyl-18,19,20-trinor-5,8(9),13-prostadiensäuremethylester (A) und
(5Z,9R,11R,13E,15S)-9-Fluor-11,15-bis-(tetrahydropyran-2-yloxy)-17-phenyl-18, 19,20-trinor-5,13-prostadiensäuremethylester (B)

816 mg (1,43 mmol) der in Beispiel 11b dargestellten Verbindung setzt man in Analogie zu Beispiel 1a um und isoliert nach Aufarbeitung und Reinigung 247 mg (447 µmol, 31%) der Titelverbindung A sowie 314 mg (549 µmol, 38%) der Titelverbindung (B).
- IR (Film) von A:: 3090, 3060, 3020, 2940, 2850, 2830, 1740, 1605, 1440, 1355, 1205, 1135, 1120, 1080, 1030, 980, 750 und 705 cm⁻¹.
- IR (Film) von B:: 3090, 3060, 3010, 2950, 2870, 1740, 1605, 1500, 1455, 1440, 1365, 1355, 1200, 1135, 1080, 1020, 870, 820, 750 und 700 cm⁻¹.

### Beispiel 11b

(5Z,9S,11R,13E,15S)-9-Hydroxy-11,15-bis-(tetrahydropyran-2-yloxy)-17-phenyl-18,19,20-trinor-5,13-prostadiensäuremethylester

1,12 mg (1,66 mmol) der in Beispiel 11c dargestellten Verbindung setzt man in Analogie zu Beispiel 9b um und isoliert nach Aufarbeitung und Reinigung 816 mg (1,43 mmol, 86%) der Titelverbindung als farbloses Öl.
- IR (Film):: 3600-3200, 3090, 3060, 3010, 2950, 2870, 1735, 1605, 1445, 1440, 1200, 1135, 1115, 1080, 1025, 975, 870, 815, 750 und 700 cm⁻¹.

### Beispiel 11c

(5Z,9S,11R,13E,15S)-9-Benzoyloxy-11,15-bis-(tetrahydropyran-2-yloxy)-17-phenyl-18,19,20-trinor-5,13-prostadiensäuremethylester

1,11 g (1,88 mmol) der in Beispiel 9d dargestellten Verbindung setzt man in Analogie zu Beispiel 9c um und isoliert nach Aufarbeitung und Reinigung 1,12 g (1,66 mmol, 88%) der Titelverbindung als farbloses Öl.
- IR (Film):: 3090, 3060, 3030, 3010, 2940, 2870, 1740, 1720, 1600, 1585, 1450, 1440, 1355, 1315, 1375, 1120, 1025, 970, 870. 810, 750, 710 und 700 cm⁻¹.

### Beispiel 12

(5Z,11R,13E,15S)-11,15-Dihydroxy-17-phenyl-18,19,20-trinor-5,8(9),13-prostatriensäure

165 mg (428 µmol) der in Beispiel 11 dargestellten Verbindung setzt man in Analogie zu Beispiel 2 um und isoliert nach Aufarbeitung 63 mg (170 µmol, 40%) der Titelverbindung als farbloses Öl.
- IR (Film):: 3600-2400, 3090, 3060, 3030, 2930, 2860, 1710, 1605, 1500, 1455, 1410, 1245, 1050, 975, 750 und 700 cm⁻¹.

## Patentansprüche

1. Δ⁸- -Prostaglandin-Derivate der Formel I, 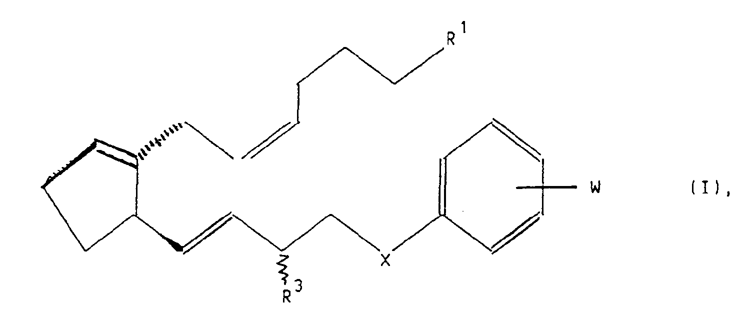 worin 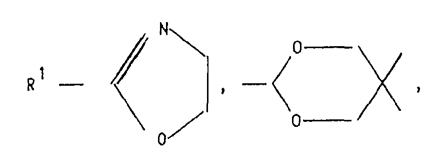 COOR⁴, wobei R⁴ Wasserstoff oder oder einen gegebenenfalls durch Halogen, Phenyl, C₁-C₄-Alkoxy oder Di-(C₁-C₄)-alkylamino substituierten -C₁-C₁₀-Alkyl-Rest, einen C₃-C₁₀-Cycloalkyl-, einen C₇-C₁₆-Aralkyl-, einen durch W substituierten Phenacyl- Rest, einen C₆-C₁₂-Aryl-Rest oder einen 5- oder 6-gliedrigen heterocyclischen Rest mit wenigstens einem N-, O- oder S-Atom bedeuten kann, oder R¹ ein CONHR⁵-Rest mit R⁵ in der Bedeutung Wasserstoff, C₁-C₁₀-Alkanoyl oder C₁-C₁₀-Alkansulfonyl sein kann,
R³ ein Wasserstoffatom oder eine freie oder funktionell abgewandelte Hydroxygruppe, wobei die OH-Gruppe jeweils α- oder β-ständig sein kann, bedeuten,
X eine CH₂-Gruppe. ein O- oder S-Atom,
W Wasserstoff, -OR⁶, Halogen, -CN-, -NO₂, Trifluormethyl oder COOR⁶,
R⁶ Wasserstoff, C₁-C₁₀-Alkyl, durch Halogen substituiertes C₆-C₁₂-Aryl oder C₇-C₁₆-Aralkyl sein kann und, falls R⁴ Wasserstoff bedeutet, deren Salze mit physiologisch vertraglichen Basen, sowie die α-, β- oder γ-Cyclodextrinclathrate, sowie die mit Liposomen verkapselten Verbindungen der Formel I bedeuten.

2. Δ⁸-Prostaglandin-Derivate der Formel I nach Anspruch 1, dadurch gekennzeichnet, daß
R¹ den Rest COOR⁴ mit R⁴ als Wasserstoff oder C₁-C₆-Alkyl oder R¹ den Rest CONHR⁵ mit R⁵ als Methylsulfonyl,
X eine CH₂-Gruppe oder ein O-Atom,
R³ Wasserstoff oder Hydroxy und
W Wasserstoff oder Fluor bedeuten.

3. Verfahren zur Herstellung der Verbindungen der Formel IA, 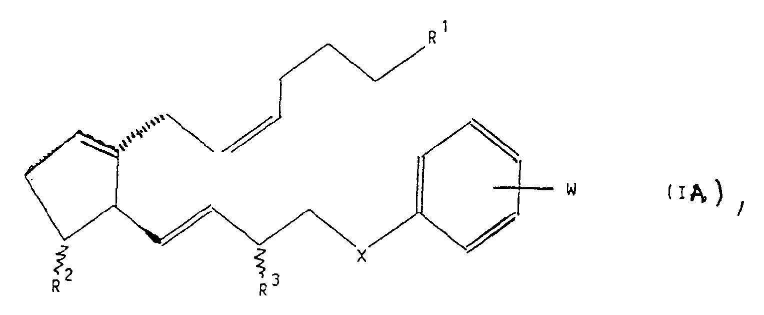 worin 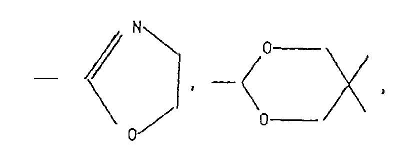 COOR⁴, wobei R⁴ Wasserstoff oder
oder einen gegebenenfalls durch Halogen, Phenyl, C₁-C₄-Alkoxy oder Di-(C₁-C₄)-alkylamino substituierten C₁-C₁₀-Alkyl-Rest, einen C₃-C₁₀-Cycloalkyl-, einen C₇-C₁₆-Aralkyl-, einen durch W substituierten Phenacyl-Rest, einen C₆-C₁₂-Aryl-Rest oder einen 5- oder 6-gliedrigen heterocyclischen Rest mit wenigstens einem N- , O- oder S-Atom bedeuten kann, oder R¹ ein CONHR⁵-Rest mit R⁵ in der Bedeutung Wasserstoff, C₁-C₁₀-Alkanoyl oder C₁-C₁₀-Alkansulfonyl sein kann,
R² und R³ jeweils ein Wasserstoffatom oder eine freie oder funktionell abgewandelte Hydroxygruppe, wobei die OH-Gruppe jeweils α- oder β-ständig sein kann, bedeuten,
X eine CH₂-Gruppe, ein O- oder S-Atom,
W Wasserstoff, -OR⁶, Halogen, -CN-, NO₂, Trifluormethyl oder COOR⁶,
R⁶ Wasserstoff, C₁-C₁₀-Alkyl, durch Halogen substituiertes C₆-C₁₂-Aryl oder C₇-C₁₆-Aralkyl sein kann und, falls R⁴ Wasserstoff bedeutet, deren Salze mit physiologisch verträglichen Basen, sowie die α-, β- oder γ-Cyclodextrinclathrate, sowie die mit Liposomen verkapselten Verbindungen der Formel IA bedeuten.
dadurch gekennzeichnet, daß man eine Verbindung der Formel II 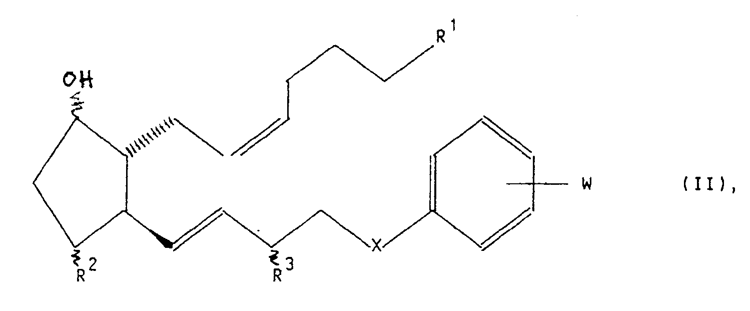 worin
R¹, R², R³, X und W die oben angegebenen Bedeutungen aufweisen und freie OH-Gruppen in R², R³ und W geschützt sind, mit Diethylaminoschwefeltrifluorid oder (HF)ₙ-Pyridin oder SeF₄, Pyridin umsetzt und geschützte Hydroxygruppen in R², R³ und W freisetzt und/oder freie Hydroxygruppen verestert, verethert und/oder eine veresterte Carboxygruppe verseift oder eine Carboxylgruppe mit einer physiologisch verträglichen Base in ein Salz überführt oder mit α-, β- oder γ-Cyclodextrin zu einem Clathrat umsetzt oder mit Liposomen verkapselt.

4. Arzneimittel enthaltend eine oder mehrere Verbindungen der Formel I nach Anspruch 1 und übliche Hilfs-, Träger- und Zusatzstoffe.

5. Verwendung der Verbindungen der Formel IA nach Anspruch 3 zur Herstellung eines Arzneimittels zur Behandlung von Krankheiten, die auf einer Störung der TXA₂/PGH₂-Bildung beruhen.

6. Verwendung der Verbindungen der Formel IA nach Anspruch 3 zur Herstellung von Diagnostika zur Charakterisierung von Prostaglandinrezeptoren und von TXA₂/PGH₂-Rezeptorsubtypen.

## Claims

1. Δ⁸-prostaglandin derivatives of formula I 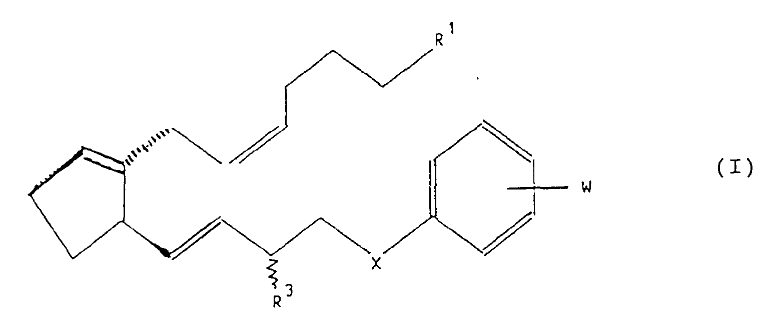 in which
R¹ may be 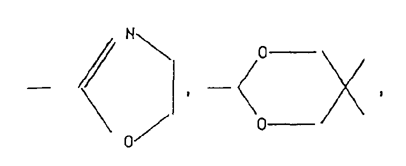 COOR⁴, wherein R⁴ may represent hydrogen or a C₁-C₁₀-alkyl radical that is optionally substituted by halogen, phenyl, C₁-C₄-alkoxy or by di-(C₁-C₄)-alkylamino, a C₃-C₁₀-cycloalkyl radical, a C₇-C₁₆-aralkyl radical, a phenacyl radical substituted by W, a C₆-C₁₂-aryl radical or a 5- or 6-membered heterocyclic radical having at least one N, O or S atom, or R¹ may be a CONHR⁵ radical wherein R⁵ represents hydrogen, C₁-C₁₀-alkanoyl or C₁-C₁₀-alkanesulphonyl,
R³ represents a hydrogen atom or a free or functionally modified hydroxy group, it being possible in each case for the OH group to be in the α- or β-configuration,
X represents a CH₂ group, an O atom or an S atom,
W represents hydrogen, -OR⁶, halogen, -CN-, -NO₂, trifluoromethyl or COOR⁶,
R⁶ represents hydrogen, C₁-C₁₀-alkyl, halo-substituted C₆-C₁₂-aryl or C₇-C₁₆-aralkyl,
and, when R⁴ represents hydrogen, the salts thereof with physiologically tolerable bases, as well as the α-, β- or γ-cyclodextrin clathrates, as well as the compounds of formula I encapsulated with liposomes.

2. Δ⁸-prostaglandin derivatives of formula I according to claim 1, characterised in that
R¹ represents the radical COOR⁴ wherein R⁴ is hydrogen or C₁-C₆-alkyl, or R¹ represents the radical CONHR⁵ wherein R⁵ is methylsulphonyl,
X represents a CH₂ group or an O atom,
R³ represents hydrogen or hydroxy, and
W represents hydrogen or fluorine.

3. Process for the preparation of the compounds of formula IA 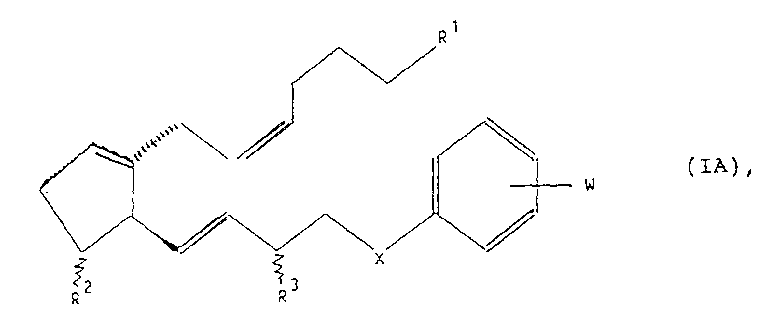 in which
R¹ may be 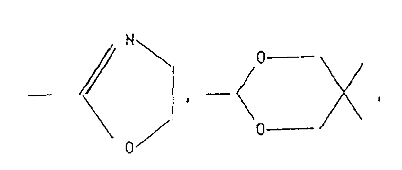 COOR⁴, wherein R⁴ may represent hydrogen or a C₁-C₁₀-alkyl radical that is optionally substituted by halogen, phenyl, C₁-C₄-alkoxy or by di-(C₁-C₄)-alkylamino, a C₃-C₁₀-cycloalkyl radical, a C₇-C₁₆-aralkyl radical, a phenacyl radical substituted by W, a C₆-C₁₂-aryl radical or a 5- or 6-membered heterocyclic radical having at least one N, O or S atom, or R¹ may be a CONHR⁵ radical wherein R⁵ represents hydrogen, C₁-C₁₀-alkanoyl or C₁-C₁₀-alkanesulphonyl,
each of R² and R³ represents a hydrogen atom or a free or functionally modified hydroxy group, it being possible in each case for the OH group to be in the α- or β-configuration,
X represents a CH₂ group, an O atom or an S atom,
W represents hydrogen, -OR⁶, halogen, -CN-, -NO₂, trifluoromethyl or COOR⁶,
R⁶ represents hydrogen, C₁-C₁₀-alkyl, halo-substituted C₆-C₁₂-aryl or C₇-C₁₆-aralkyl,
and, when R⁴ represents hydrogen, the salts thereof with physiologically tolerable bases, as well as the α-, β- or γ-cyclodextrin clathrates, as well as the compounds of formula IA encapsulated with liposomes,
characterised in that a compound of formula II 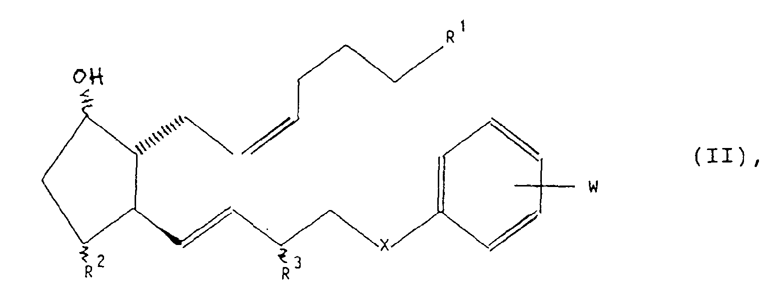 in which R¹, R², R³, X and W have the meanings given above and free OH groups in R², R³ and W are protected, is reacted with diethylaminosulphur trifluoride or (HF)ₙ-pyridine or SeF₄-pyridine and protected hydroxy groups in R², R³ and W are freed and/or free hydroxy groups are esterified or etherified and/or an esterified carboxy group is hydrolysed or a carboxy group is converted into a salt with a physiologically tolerable base or is reacted with α-, β- or γ-cyclodextrin to form a clathrate or is encapsulated with liposomes.

4. Medicament containing one or more compounds of formula I according to claim 1 and customary adjuvants, carriers and additives.

5. Use of the compounds of formula IA according to claim 3 in the preparation of a medicament for the treatment of disorders based on a disturbance in TXA₂/PGH₂ formation.

6. Use of the compounds of formula IA according to claim 3 in the preparation of diagnostic agents for the characterisation of prostaglandin receptors and of TXA₂/PGH₂ receptor sub-types.

## Revendications

1. Δ⁸-prostaglandines de formule I ci-dessous : 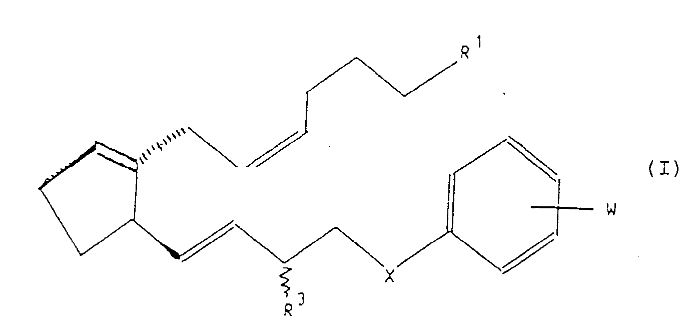 dans laquelle
R¹ est un groupe 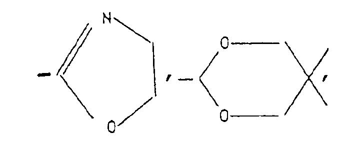 COOR⁴, R⁴ étant l'hydrogène,
ou un alkyle en C₁-C₁₀ éventuellement substitué par un halogène, un phényle, un alcoxy en C₁-C₄ ou un groupe di-(C₁-C₄)-alkylamino, un cycloalkyle en C₃-C₁₀, un aralkyle en C₇-C₁₆, un phénacyle substitué par W, un aryle en C₆-C₁₂ ou un radical hétérocyclique à 5 ou 6 chaînons et au moins un atome d'azote, d'oxygène ou de soufre, ou encore R¹ est un radical CONHR⁵, R⁵ pouvant être l'hydrogène, un alcanoyle en C₁-C₁₀ ou un alcane-sulfonyle en C₁-C₁₀,
R³ est un atome d'hydrogène ou un groupe hydroxylique libre ou fonctionnellement modifié, le groupe OH pouvant être toujours à la position α ou β,
X est un groupe CH₂ ou bien un atome d'oxygène ou de soufre et
W l'hydrogène, un groupe -OR⁶, un halogène ou un groupe -CN, -NO₂, trifluorométhyle ou COOR⁶,
R⁶ représentant l'hydrogène, un alkyle en C₁-C₁₀, un aryle en C₆-C₁₂ halogéné ou un aralkyle en C₇-C₁₆, ainsi que, si R⁴ est l'hydrogène, les sels de ces composés formés avec des bases physiologiquement compatibles et tolérées, leur clathrates formés avec la cyclodextrine α, β ou γ, et aussi les composés de formule I encapsulés avec des liposomes.

2. Δ⁸-prostaglandines de formule I selon la revendication 1, caractérisées en ce que
R¹ est un groupe COOR⁴, R⁴ étant l'hydrogène ou un alkyle en C₁-C₆, ou bien R¹ est un radical CONHR⁵, R⁵ étant le radical méthyl-sulfonyle,
X est un groupe CH₂ ou un atome d'oxygène,
R³ l'hydrogène ou le groupe hydroxy et
W l'hydrogène ou le fluor.

3. Procédé de préparation des composés de formule IA : 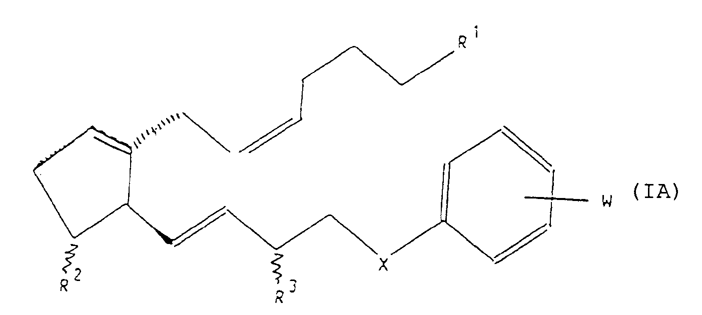 dans laquelle
R¹ est un groupe 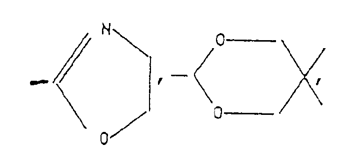 COOR⁴, R⁴ étant l'hydrogène,
ou un alkyle en C₁-C₁₀ éventuellement substitué par un halogène, un phényle, un alcoxy en C₁-C₄ ou un groupe di-(C₁-C₄)-alkylamino, un cycloalkyle en C₃-C₁₀, un aralkyle en C₇-C₁₆, un phénacyle substitué par W, un aryle en C₆-C₁₂ ou un radical hétérocyclique à 5 ou 6 chaînons et au moins un atome d'azote, d'oxygène ou de soufre, ou encore R¹ est un radical CONHR⁵, R⁵ pouvant être l'hydrogène, un alcanoyle en C₁-C₁₀ ou un alcanesulfonyle en C₁-C₁₀,
R² et R³ sont chacun un atome d'hydrogène ou un groupe hydroxylique libre ou fonctionnellement modifié, le groupe OH pouvant être à la position α ou β,
X est un groupe CH₂ ou bien un atome d'oxygène ou de soufre et
W l'hydrogène, un groupe -OR⁶, un halogène ou un groupe -CN, -NO₂, trifluorométhyle ou COOR⁶,
R⁶ représentant l'hydrogène, un alkyle en C₁-C₁₀, un aryle en C₆-C₁₂ halogéné ou un aralkyle en C₇-C₁₆, ainsi que, si R⁴ est l'hydrogène, les sels de ces composés formés avec des bases physiologiquement compatibles et tolérées, leur clathrates formés avec la cyclodextrine α, β ou γ, et aussi les composés de formule IA,
procédé caractérisé en ce que l'on fait réagir un composé de formule II : 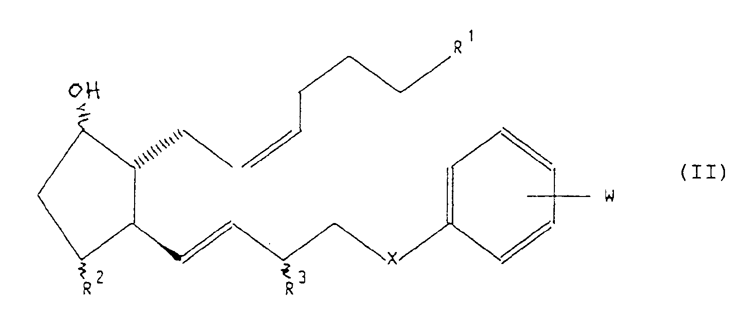 dans laquelle les divers symboles ont les significations ci-dessus indiquées et des groupes OH libres de deux R², R³ et W sont protégés, avec du diéthanylaminosulfotrifluorure ou un composé (HF)ₙ-pyridine ou SeF⁴-pyridine, et on libère des hydroxyles protégés de R², R³ et W et/ou on estérifie ou éthérifie des hydroxyles libres et/ou on saponifie un groupe carboxylique estérifié ou bien on transforme un groupe carboxylique en un sel avec une base physiologiquement compatible ou encore on forme un clathrate avec la cyclodextrine α, β ou γ, ou on encapsule avec des liposomes.

4. Médicaments contenant un ou plusieurs composés de formule I selon la revendication 1 avec des adjuvants, véhicules et additifs courants.

5. Emploi des composés de formule IA de la revendication 3 pour en préparer des médicaments destinés au traitement de maladies dues à une perturbation de la formation de TXA₂/PGH₂.

6. Emploi des composés de formule IA de la revendication 3 pour en préparer des diagnostics destinés à caractériser des récepteurs de prostaglandines et des types de récepteurs de TXA₂/PGH₂.
